# EUROPÄISCHE PATENTSCHRIFT

(11) **EP 3 369 371 B1**
(45) Veröffentlichungstag und Bekanntmachung des Hinweises auf die Patenterteilung: **18.09.2024**
(21) Anmeldenummer: 18159453.2
(22) Anmeldetag: 01.03.2018
(51) Int. Cl.: A61B 5/00

(54) **MULTISENSORDRESS UND DEREN VERWENDUNG**
GARMENT WITH MULTIPLE SENSORS AND THE USE OF IT
VÊTEMENT AVEC MULTIPLES CAPTEURS ET SON UTILISATION

(30) Priorität: 02.03.2017 DE 102017203447
(43) Veröffentlichungstag der Anmeldung: 05.09.2018
(73) Patentinhaber: Star Cooperation GmbH, 71034 Böblingen (DE)
(72) Erfinder: Flemming, André, 71116 Gärtringen (DE); Stegmann, Tim, 96052 Bamberg (DE)
(74) Vertreter: Kohler Schmid Möbus Patentanwälte

(56) Entgegenhaltungen:
- EP-A1- 0 714 263
- EP-A2- 2 505 090
- EP-B1- 0 714 263
- US-A1- 2005 034 485
- US-A1- 2015 075 303
- US-A1- 2016 095 527

## Beschreibung

### Hintergrund der Erfindung

Die Erfindung betrifft eine Bekleidung in Form eines Multisensordresses, d.h. eine Bekleidung zur Datenerfassung mit einem Grundkörper aus einem flexiblen Material, mehreren Sensoranordnungen und einer Datensammeleinheit, die mit zumindest einigen Sensoranordnungen verbunden ist. Die Erfindung betrifft weiterhin die Verwendung einer solchen Bekleidung.

Es ist bekannt, Bekleidungen mit Sensoren zu versehen, um die Zustände ihrer Benutzer, insbesondere durch Lagesensoren, zu erfassen.

In D2 (US2005/0034485A1) wird ein Kleidungsstück mit biometrischen Sensoren beschrieben, das zur medizinischen Überwachung von Personen dient. Die Sensoren sind mittels eines Datenübertragungs- und Energieversorgungsbusses verbunden. Weitere Kleidungsstücke mit biometrischen Sensoren sind in EP 2 505 090 A2 und in US 2016/0095527 A1 offenbart.

Die US 8,032,199 B2 offenbart eine Bekleidung mit dehnbaren Bereichen und Gürteln, sodass Sensoranordnungen der Bekleidung den Bewegungen des Benutzers folgen.

Aus der US 2010/0256475 A1 ist eine Bekleidung mit einer Sensoranordnung bekannt geworden, die mittels einer elektrischen Verbindungsleitung mit einer Datensammeleinheit verbindbar ist, wobei die Verbindungsleitung eine Druckknopfverbindung aufweist. Das Lösen der Druckknopfverbindung erleichtert die Reinigung der Bekleidung.

Die US 2008/0001735 A1 offenbart eine Bekleidung mit mehreren Sensoranordnungen, die drahtlos mit einer Auswerteeinheit kommunizieren kann.

Aus der US 9,285,788 B2 ist eine Bekleidung mit mehreren Sensoranordnungen bekannt geworden. Mehrere Sensoranordnungen sind lokal mit einem Prozessor verbunden, der eine Auswertung der erfassten Daten vornimmt.

Die bekannte Bekleidung zur Erfassung von Zuständen eines Benutzers sind aufgrund ihrer Verkabelung der Sensoranordnungen nur aufwändig an- und auszuziehen. Weiterhin ist die Verkabelung störend beim Tragen der Bekleidung. Andere Arten bekannter Bekleidung sind sehr aufwändig konstruiert und daher sehr teuer.

### Aufgabe der Erfindung

Es ist daher Aufgabe der Erfindung, eine Bekleidung bereit zu stellen, die kosteneffektiv herstellbar und dennoch komfortabel zu tragen ist. Es ist weiterhin Aufgabe der Erfindung, eine entsprechende Verwendung der Bekleidung anzugeben.

### Beschreibung

Diese Aufgabe wird erfindungsgemäß gelöst durch eine Bekleidung gemäß Patentanspruch 1 und eine Verwendung gemäß Patentanspruch 11.

Die Unteransprüche geben bevorzugte Weiterbildungen wieder.

Die Aufgabe wird somit gelöst durch eine Bekleidung zur Datenerfassung, wobei die Bekleidung einen Grundkörper aus einem flexiblen Material und mehrere Sensoranordnungen aufweist. Die Sensoranordnungen weisen jeweils mindestens einen Sensor zur Zustandserfassung des Benutzers auf. Die Bekleidung umfasst weiterhin eine Datensammeleinheit, die mit zumindest einigen Sensoranordnungen elektrisch verbunden ist, um die von den Sensoranordnungen erfassten Daten zu sammeln. Die Bekleidung weist eine Busleitung auf, auf der zumindest einige der Sensoranordnungen angeordnet sind, wobei die Busleitung mit der Datensammeleinheit elektrisch verbunden ist, um von den Sensoranordnungen erfasste Daten an die Datensammeleinheit zu übermitteln. Die Busleitung weist einen flexiblen Trägerstreifen mit mehreren flexiblen elektrischen Leitungen zur elektrischen Verbindung zumindest einiger Sensoranordnungen mit der Datensammeleinheit auf.

Der Grundkörper kann in Form eines Kleidungsstücks zur Bedeckung einer Gliedmaße, insbesondere in Form einer Hose, eines Ärmels oder eines Handschuhs, ausgebildet sein. Der Grundkörper kann in Form von Unterwäsche ausgebildet sein. Der Grundkörper ist jedoch vorzugsweise in Form eines Overalls oder eines Jumpsuits ausgebildet. Hierdurch kann die Bekleidung im Arbeitsalltag bei Bedarf getragen werden, ohne dass sich der Benutzer weitgehend umziehen muss.

Der Grundkörper kann in Form eines Gurts, insbesondere in Form eines Hüftgurts oder Brustgurts, ausgebildet sein. Die Bekleidung kann hierdurch besonders gut zur Erkennung von Haltungsfehlern des Benutzers der Bekleidung eingesetzt werden.

Der zumindest eine Sensor der Sensoranordnungen kann in Form eines Temperatursensors, eines Pulssensors, eines Feuchtigkeitssensors, eines Lagesensors, eines Beschleunigungssensors, eines Drehratensensors, eines Gassensors, eines Schallsensors, eines Lichtsensors, eines Drucksensors, insbesondere in Form eines Luftdrucksensors, eines Strahlungssensors, insbesondere in Form eines Röntgenstrahlensensors oder Sensors für radioaktive Strahlung, oder eines Magnetfeldsensors ausgebildet sein. Vorzugsweise ist der Sensor in Form einer inertialen Messeinheit (Inertial Movement Unit; IMU) ausgebildet.

Zumindest eine Sensoranordnung kann einem Hauptknochen des Benutzers der Bekleidung zugeordnet sein. Vorzugsweise sind mehrere Sensoranordnungen jeweils einem Hauptknochen des Benutzers der Bekleidung zugeordnet. Hierdurch sind die Bewegungen des Benutzers, und insbesondere dessen Haltungsschäden, besonders präzise detektierbar.

Zusätzlich zu zumindest einem Sensor kann die Sensoranordnung einen Mikrocontroller und/oder einen Transceiver aufweisen. Der Mikrocontroller kann dazu ausgebildet sein, mit dem zumindest einen Sensor zu kommunizieren und die von ihm gemessenen Daten bereit zu stellen. Der Transceiver kann dazu ausgebildet sein, die Daten über die Busleitung zu versenden.

Die Busleitung weist mehrere elektrische Leitungen auf, die mit der Sensoranordnung in elektrischem Kontakt stehen. Dabei wird generell im Rahmen dieser Erfindung "elektrisch" mit "elektronisch" gleichgesetzt.

Die flexiblen elektrischen Leitungen sind auf einem flexiblen Trägerstreifen der Busleitung angeordnet. Hierdurch wird der Tragekomfort der Bekleidung signifikant erhöht. Die elektrischen Leitungen können dabei in den Trägerstreifen eingenäht, eingestrickt, eingewirkt, eingehäkelt und/oder eingewoben sein. Alternativ oder zusätzlich dazu können die elektrischen Leitungen in einen Grundkörper der Bekleidung eingenäht, eingestrickt, eingewirkt, eingehäkelt und/oder eingewoben sein. Der Trägerstreifen kann Teil des Grundkörpers sein oder in Form eines zusätzlichen Trägerstreifens auf dem Grundkörper angeordnet sein.

Zumindest eine elektrische Leitung kann gebogene Bereiche aufweisen, sodass eine Flexibilität dieser elektrischen Leitung auch in Längsrichtung des Trägerstreifens gewährbar ist. Alternativ oder zusätzlich dazu kann der Trägerstreifen in dessen Längsrichtung elastisch ausgebildet sein. Wenn sowohl der Trägerstreifen als auch die elektrischen Leitungen der Busleitung in Längsrichtung der Busleitung elastisch dehnbar ausgebildet sind, wird ein weiter erhöhter Tragekomfort erzielt.

Die elektrischen Leitungen der Busleitung können zur elektrisch parallelen Anbindung mehrerer Sensoranordnungen, insbesondere aller Sensoranordnungen, der Busleitung ausgebildet sein. Hierdurch fallen keine weiteren Sensoranordnungen aus, wenn eine Sensoranordnung defekt und/oder unzureichend kontaktiert ist.

Der Trägerstreifen ist vorzugsweise aus Stoff ausgebildet. Der Trägerstreifen kann auf den Grundkörper genäht sein.

Alternativ oder zusätzlich dazu kann der Grundkörper aus Stoff ausgebildet sein. Der Grundkörper kann aus mehreren Stoffabschnitten zusammengesetzt, insbesondere zusammengenäht, sein.

In besonders bevorzugter Ausgestaltung der Erfindung ist/sind zumindest eine Sensoranordnung, insbesondere mehrere Sensoranordnungen, vorzugsweise alle Sensoranordnungen, reversibel lösbar, d.h. zerstörungsfrei, mit einer Busleitung verbunden. Zumindest eine Sensoranordnung, insbesondere mehrere Sensoranordnungen, vorzugsweise alle Sensoranordnungen kann/können werkzeugfrei lösbar mit einer Busleitung verbunden sein.

Weiter bevorzugt ist/sind zumindest eine Sensoranordnung, insbesondere mehrere Sensoranordnungen, vorzugsweise alle Sensoranordnungen nur durch Anpressdruck auf die jeweilige Sensoranordnung elektrisch mit den elektrischen Leitungen einer Busleitung verbunden. Der Anpressdruck kann durch eine Tasche, ein Band, einen Gurt oder dergleichen der Bekleidung erzeugt werden.

Die Bekleidung kann eine Kabelschlaufe aufweisen, in der zumindest eine Busleitung zumindest abschnittsweise relativ zum Grundkörper bewegbar geführt ist.

Um die Modularität der Bekleidung und damit deren Anpassbarkeit an bestimmte Verwendungen zu erhöhen, kann/können zumindest eine Sensoranordnung, insbesondere mehrere Sensoranordnungen, vorzugsweise alle Sensoranordnungen, jeweils in einer, insbesondere flexiblen, Tasche aufgenommen sein.

Die zuvor beschriebenen Anordnungsmöglichkeiten einer Sensoranordnung an einer Busleitung können analog für die Datensammeleinheit realisiert sein.

Die zuvor beschriebenen Anordnungsmöglichkeiten einer Sensoranordnung und/oder einer Datensammeleinheit an einer Busleitung werden auch unabhängig von einer Bekleidung zur Datenerfassung als eigenständige Erfindung angesehen.

Weiter bevorzugt weist die Bekleidung mehrere Busleitungen auf. Die Busleitungen können in einem Knotenpunkt, insbesondere in Form der Datensammeleinheit, zusammengeführt werden.

Vorzugsweise sind mehrere Sensoranordnungen, insbesondere alle Sensoranordnungen, verschiedener Busleitungen parallel geschaltet. In diesem Fall sind elektrisch gleichartige elektrische Leitungen der Busleitungen im Knotenpunkt miteinander verbunden. Beispielsweise können alle elektrischen Leitungen der Busleitungen, die von der Datensammeleinheit mit einer Versorgungsspannung für die Sensoranordnungen gespeist werden, parallel geschaltet werden.

Die Datensammeleinheit kann eine Batterie und/oder einen Akkumulator aufweisen, um die meisten elektrischen Komponenten der Bekleidung, insbesondere alle elektrischen Komponenten der Bekleidung, mit Spannung zu versorgen.

Die Bekleidung kann eine Auswerteeinheit aufweisen. Die Datensammeleinheit kann dazu ausgebildet sein, mit der Auswerteeinheit, insbesondere drahtlos, zu kommunizieren. Vorzugsweise ist die Datensammeleinheit dazu ausgebildet, die von den Sensoranordnungen der Bekleidung gemessenen Daten nur zu kommunizieren und zu speichern, nicht aber, diese Daten auszuwerten. Hierdurch kann die Datensammeleinheit schlicht und kompakt ausgebildet werden. Die Datensammeleinheit kann getrennt von der Auswerteeinheit angeordnet sein. Alternativ dazu sind die Datensammeleinheit und die Auswerteeinheit in einem gemeinsamen Gehäuse angeordnet und/oder in einer gemeinsamen elektrischen Anordnung vorgesehen.

Die Bekleidung weist in bevorzugter Ausgestaltung der Erfindung eine akustische, optische und/oder sensorische Feedbackeinrichtung auf. Die Feedbackeinrichtung ist mit der Datensammeleinheit und/oder der Auswerteeinheit verbunden. Die Feedbackeinrichtung kann dazu ausgebildet sein, bei bestimmten Zuständen der Bekleidung eine Rückmeldung an den Benutzer zu geben. Die Feedbackeinrichtung kann dem Benutzer dadurch insbesondere eine Rückmeldung bei einem Haltungsfehler, d.h. einer Fehlhaltung des Benutzers, geben. Die Feedbackeinrichtung weist dabei bevorzugt eine Vibrationseinheit auf, die dem Benutzer eine sensorische Rückmeldung geben kann. Die Vibrationseinheit kann einen elektrischen Motor mit einer Unwucht aufweisen.

Die Bekleidung kann ein Holster zur Aufnahme der Datensammeleinheit und/oder der Auswerteeinheit aufweisen.

Die Bekleidung weist erfindungsgemäß eine Virtual Reality Brille (VR-Brille) auf. Eine Datenverarbeitungsvorrichtung der Virtual Reality Brille ist erfindungsgemäß dabei dazu ausgebildet, Daten der Datensammeleinheit, insbesondere drahtlos, zu empfangen und zu verarbeiten. Die Datenverarbeitungsvorrichtung kann einen Mikroprozessor aufweisen. Die Datenverarbeitungsvorrichtung kann einen Computer, ein Field Programmable Gate Array (FPGA), ein System-On-Chip, einen Mikrocontroller oder einen Mikrocomputer aufweisen. Die Datenverarbeitungsvorrichtung kann in Form eines Smartphones ausgebildet sein.

Die Bekleidung kann einen Nahfeldkommunikationsempfänger aufweisen, um zumindest eine Nahfeldkommunikationseinheit in der Umgebung der Bekleidung zu detektieren. Weiterhin kann die Bekleidung einen Nahfeldkommunikationssender aufweisen.

Der Nahfeldkommunikationsempfänger kann einen Teil einer Datenverarbeitungsvorrichtung einer VR-Brille darstellen.

Den Nahfeldkommunikationseinheiten können Informationen zugewiesen sein, die durch die VR-Brille darstellbar sind. Weiterhin können die Nahfeldkommunikationsempfänger, insbesondere durch eine Messung der Signallaufzeiten von den Nahfeldkommunikationseinheiten zum Nahfeldkommunikationsempfänger, die räumliche Orientierung im Rahmen der Benutzung der VR-Brille erleichtern.

Die zuvor beschriebenen Nahfeldkommunikationsmerkmale werden auch unabhängig von einer Bekleidung zur Datenerfassung als eigenständige Erfindung angesehen.

Die Erfindung betrifft weiterhin ein Verfahren zum Betrieb einer zuvor beschriebenen Bekleidung. Dabei werden bevorzugt die Daten einer ersten Sensoranordnung auf einer ersten Busleitung von der Datensammeleinheit abgefragt und anschließend die Daten einer zweiten Sensoranordnung auf der ersten Busleitung von der Datensammeleinheit abgefragt. Zwischen dem Abfragen der Daten der ersten Sensoranordnung und dem Abfragen der Daten der zweiten Sensoranordnung kann von der Datensammeleinheit eine Pause eingehalten werden, die größer ist als die benötigte Zeit zur Abfrage der Daten der ersten Sensoranordnung oder der zweiten Sensoranordnung. Bevorzugt ist die Pause größer als 500µs, insbesondere größer als 700µs, besonders bevorzugt größer als 900µs. Hierdurch kann zumindest eine weitere Sensoranordnung auf der ersten Busleitung ergänzt werden, ohne die benötigte Gesamtzeit zur Abfrage aller Sensoranordnungen auf der ersten Busleitung zu erhöhen.

Die Erfindung betrifft weiterhin eine oder mehrere Verwendungen der zuvor beschriebenen Bekleidung:
A) Zur Programmierung des Bewegungsablaufs eines Roboters;
B) Zur Aufnahme des Bewegungsablaufs eines Arbeiters oder Sportlers;
C) Zur Überwachung des Gesundheitszustands pflege- und/oder betreuungsbedürftiger Benutzer.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1: zeigt eine schematische Draufsicht auf eine Bekleidung in Form eines Overalls.
- Fig. 2: zeigt ein teilweises schematisches Schaltbild von Busleitungen und Sensoranordnungen der Bekleidung gemäß Fig. 1.
- Fig. 3: zeigt eine schematische Draufsicht auf eine mittels einer Tasche an einer Busleitung angeordnete Sensoranordnung.
- Fig. 4: zeigt eine schematische vergrößerte teilweise Schnittansicht der Fig. 3 entlang der Linie IV-IV in Fig. 3.
- Fig. 5: zeigt eine schematische Draufsicht auf einen Benutzer mit einer VR-Brille und mehreren Nahfeldkommunikationseinheiten auf einem Gegenstand.

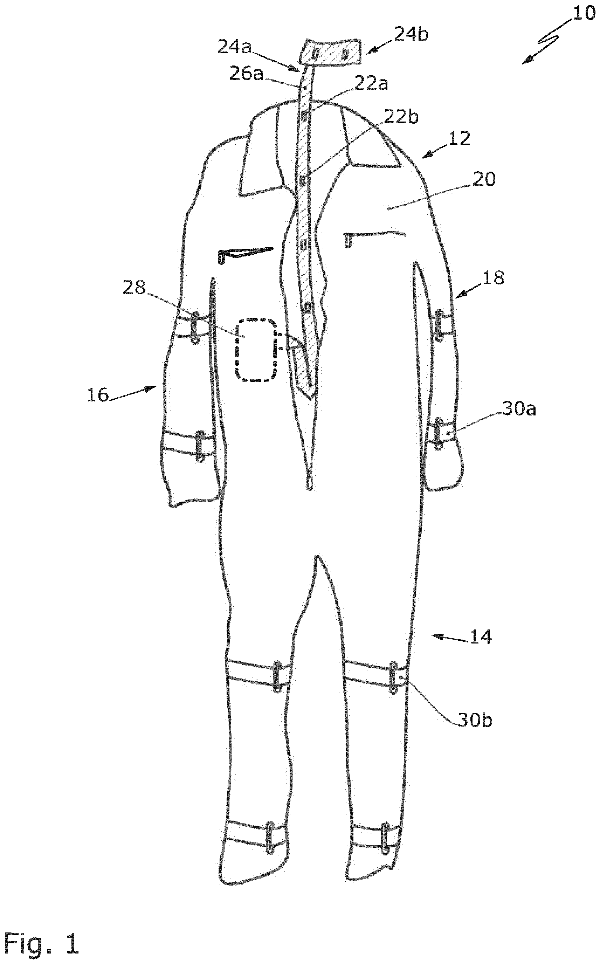

**Fig. 1** zeigt eine Bekleidung **10,** hier in Form eines Overalls. Die Bekleidung 10 hat einen Oberteilabschnitt **12,** einen Hosenabschnitt **14** sowie Ärmel **16** und **18.** Die Bekleidung 10 weist einen Grundkörper **20** aus Stoff auf. Genauer gesagt setzt sich der Grundkörper 20 aus mehreren miteinander vernähten Stoffbahnen zusammen. Der Grundkörper 20 kann gestrickt, gewirkt, gewebt und/oder gehäkelt sein.

Die Bekleidung 10 ist zur Datenerfassung ausgebildet. Hierzu weist die Bekleidung 10 mehrere Sensoranordnungen auf, von denen in Fig. 1 aus Gründen der Übersichtlichkeit nur die Sensoranordnungen **22a, 22b** mit einem Bezugszeichen versehen sind. Die Sensoranordnungen 22a, 22b sind auf einer Busleitung **24a** angeordnet. Die Busleitung 24a weist einen Trägerstreifen **26a** auf. Der Trägerstreifen 26a ist aus Stoff ausgebildet. Der Trägerstreifen 26a kann gestrickt, gewirkt, gewebt und/oder gehäkelt sein. Der Trägerstreifen 26a kann in Form eines dehnbaren Gewebebandes ausgebildet sein. Die Busleitung 24a dient als mechanischer Träger der Sensoranordnungen 22a, 22b und zur elektrischen Anbindung der Sensoranordnungen 22a, 22b.

Die Busleitung 24a ist elektrisch mit einer Datensammeleinheit **28** verbunden. Die Datensammeleinheit 28 ist im vorliegenden Fall in einer Tasche (nicht gezeigt) der Bekleidung 10 verstaut.

Die in Fig. 1 gezeigte Busleitung 24a verläuft im Bereich der Wirbelsäule des Benutzers (nicht gezeigt) der Bekleidung 10. Im in Fig. 1 gezeigten Ausführungsbeispiel ist die Busleitung 24a elektrisch und/oder mechanisch mit einer Busleitung **24b** verbunden. Die Busleitung 24b ist zur Erfassung von Daten im Kopfbereich eines Benutzers (nicht gezeigt) ausgebildet. Hierzu kann die Busleitung 24b an einer Mütze, einem Stirnband oder dergleichen befestigt sein. Alternativ oder zusätzlich dazu können Daten aus dem Kopfbereich des Benutzers (nicht gezeigt) über eine VR-Brille (nicht gezeigt) gesammelt werden.

Die Bekleidung 10 weist, insbesondere an zumindest einer Gliedmaße, zumindest einen Zurrgurt und/oder zumindest einen Spanngurt auf. Aus Gründen der Übersichtlichkeit sind in Fig. 1 nur die Zurrgurte **30a, 30b** mit einem Bezugszeichen versehen. Zurrgurte bzw. Spanngurte ermöglichen die enge Befestigung der Bekleidung 10 an dem Benutzer (nicht gezeigt), um die Zustände des Benutzers (nicht gezeigt) mittels der Sensoranordnungen 22a, 22b präzise erfassen zu können. Alternativ oder zusätzlich dazu kann zumindest ein Bereich der Bekleidung 10 elastisch ausgebildet sein, um sich an die Kontur des Benutzers (nicht gezeigt) anzuschmiegen.

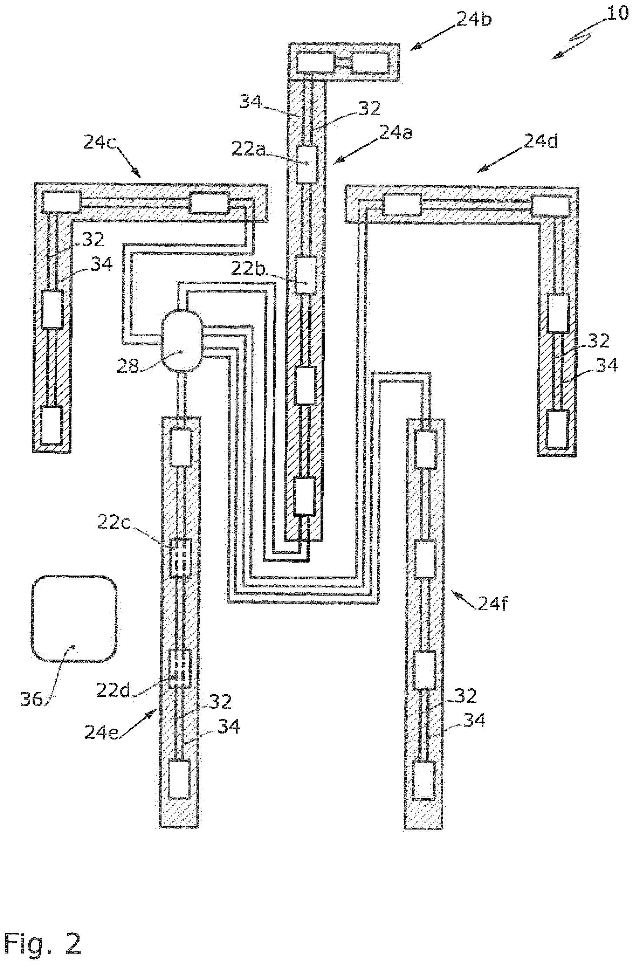

**Fig. 2** zeigt die elektrischen Komponenten der Bekleidung 10. Die Bekleidung 10 weist mehrere Busleitungen 24a, 24b, **24c, 24d, 24e** und **24f** und Sensoranordnungen auf, von denen aus Gründen der Übersichtlichkeit nur die Sensoranordnungen 22a, 22b, **22c** und **22d** mit einem Bezugszeichen versehen sind.

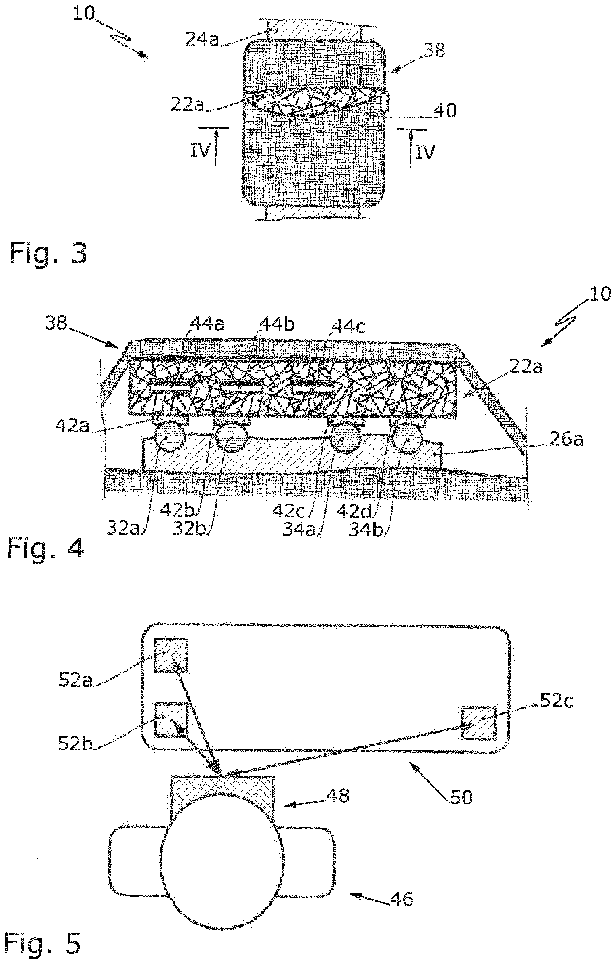

Die Busleitungen 24a-24f weisen elektrische Leitungen auf, von denen aus Gründen der Übersichtlichkeit in Fig. 2 nur die elektrischen Leitungen **32, 34** mit einem Bezugszeichen versehen sind. Die elektrische Leitung 32 steht symbolisch für die Spannungsversorgung der Busleitungen 24a-24f, die elektrische Leitung 34 steht symbolisch für die Datenkommunikation der Busleitungen 24a-24f. Tatsächlich können die Busleitungen 24a-24f (wie aus Fig. 4 ersichtlich), mehrere elektrische Leitungen zur Spannungsversorgung und/oder mehrere elektrische Leitungen zur Datenkommunikation aufweisen.

Zumindest eine elektrische Leitung 32, 34 kann in einen Gewebestreifen 26a (siehe Fig. 1) eingestrickt, eingewirkt, eingehäkelt oder eingenäht sein. Alternativ oder zusätzlich dazu kann zumindest eine elektrische Leitung 32, 34 unmittelbar in den Grundkörper 20 (siehe Fig. 1) eingestrickt, eingewirkt, eingehäkelt oder eingenäht sein. Die beschriebene Anbindung zumindest einer elektrischen Leitung 32, 34 an den Gewebestreifen 26a und/oder den Grundkörper 20 ermöglicht die elastische Ausbildung der zumindest einen elektrischen Leitung 32, 34 in deren Längserstreckung. Mit anderen Worten ist die zumindest eine elektrische Leitung 32, 34 dehnbar ausgebildet, sodass die Bekleidung 10 besonders komfortabel zu tragen ist.

Die elektrische Leitung 32 einer ersten Busleitung 24a-24f kann mit zumindest einer elektrischen Leitung 32 einer zweiten Busleitung 24a-24f elektrisch verbunden sein. Weiterhin kann die elektrische Leitung 34 einer ersten Busleitung 24a-24f mit zumindest einer elektrischen Leitung 34 einer zweiten Busleitung 24a-24f elektrisch verbunden sein. Vorzugsweise sind alle elektrisch gleichartigen Leitungen 32, 34 der Busleitungen 24a-24f elektrisch miteinander verbunden, sodass alle Sensoranordnungen 22a-22d parallel geschaltet sind.

Die elektrischen Leitungen 32, 34 können jeweils ausschließlich von einer Sensoranordnung 22a-22d zur nächsten Sensoranordnung 22a-22d ausgebildet sein, sodass die Sensoranordnungen 22a-22d der jeweiligen Busleitungen 24a-24f seriell geschaltet sind. Hierdurch kann die Gesamtlänge der elektrischen Leitungen 32, 34 verringert werden. Nachteilig an einer solchen Ausgestaltung ist der Ausfall aller Sensoranordnungen 22a-22d einer Busleitung 24a-24f ab einer defekten Sensoranordnung 22a-22d. Bevorzugt sind daher die elektrischen Leitungen 32, 34 unter den Sensoranordnungen 22a-22d durchgeführt und/oder an den Sensoranordnungen 22a-22d vorbeigeführt, wie in Fig. 2 durch strichpunktierte Linien unter den Sensoranordnungen 22c, 22d angedeutet ist. Die Sensoranordnungen 22a-22d einer Busleitung 24a-24f sind dadurch parallel geschaltet, sodass der Ausfall einer Sensoranordnung 22a-22d nicht zum Ausfall weiterer Sensoranordnungen 22a-22d führt.

Die Busleitungen 24a-24f sind, bevorzugt durch Kabel, mit der Datensammeleinheit 28 verbunden. Die Datensammeleinheit 28 bildet einen Knotenpunkt der Busleitungen 24a-24f. Die Busleitungen 24a-24f sind somit sternförmig mit der Datensammeleinheit 28 verbunden. Die Busleitungen 24a-24f können jeweils über einen Steckverbinder (nicht gezeigt) mit der Datensammeleinheit 28 reversibel lösbar verbindbar sein.

Die Datensammeleinheit 28 kann eine Spannungsversorgung (nicht gezeigt) in Form einer Batterie und/oder eines Akkumulators aufweisen, um die Busleitungen 24a-24f sowie die Sensoranordnungen 22a-22d zentral mit Spannung zu versorgen.

Die Datensammeleinheit 28 kann ein GPS-System aufweisen.

Die Datensammeleinheit 28 weist bevorzugt keine elektrischen Komponenten zur Auswertung der durch die Sensoranordnungen 22a-22d gesammelten Daten auf. Die Datensammeleinheit 28 ist demgegenüber vorzugsweise ausschließlich zur Aufnahme und Übertragung von Daten ausgebildet. Hierdurch kann die Datensammeleinheit 28 konstruktiv einfach, preisgünstig und stromsparend ausgebildet werden. Zur Datenaufnahme bzw. Datenspeicherung kann die Datensammeleinheit 28 einen Mikroprozessor aufweisen, der dazu ausgebildet ist, auf Abfragen zu reagieren und Daten bereit zu stellen. Die Datensammeleinheit 28 kann einen Computer, ein Field Programmable Gate Array (FPGA), ein System-On-Chip, einen Mikrocontroller oder einen Mikrocomputer aufweisen.

Die Datensammeleinheit 28 kann einen Datenspeicher (nicht gezeigt), insbesondere in Form einer Speicherkarte, aufweisen, um Daten der Sensoranordnungen 22a-22d sammeln und zu einem späteren Zeitpunkt übertragen zu können.

Die Datensammeleinheit 28 ist zur Datenkommunikation mit einer Auswerteeinheit **36** ausgebildet. Die Datensammeleinheit 28 kann dabei dazu ausgebildet sein, per USB-Verbindung, per ZigBee-Verbindung und/oder per Bluetooth-Verbindung mit der Auswerteeinheit 36 zu kommunizieren. Die Auswerteeinheit 36 kann Teil der Bekleidung 10 sein. Die Auswerteeinheit 36 kann einen Mikroprozessor aufweisen. Die Auswerteeinheit 36 kann einen Computer, ein Field Programmable Gate Array (FPGA), ein System-On-Chip, einen Mikrocontroller oder einen Mikrocomputer aufweisen. Die Auswerteeinheit 36 kann in Form eines Computers oder Smartphones ausgebildet sein. Die Auswerteeinheit 36 kann Teil einer VR-Brille sein.

Die Datensammeleinheit 28 kann eine Echtzeituhr (Real Time Clock; RTC) aufweisen, um eine Echtzeitdatenerfassung zu ermöglichen, wenn die Daten erst zu einem späteren Zeitpunkt übertragen werden.

**Fig. 3** zeigt einen Teil der Bekleidung 10 mit der Busleitung 24a und der Sensoranordnung 22a. Die Bekleidung 10 weist eine Tasche **38** auf, in der die Sensoranordnung 22a aufgenommen ist. Die Tasche 38 kann durch einen Verschluss **40,** hier in Form eines - geöffnet dargestellten - Reißverschlusses, geöffnet und geschlossen werden, um die Sensoranordnung 22a auszutauschen. Alternativ oder zusätzlich dazu kann der Verschluss 40 durch eine Naht oder durch einfache Überlappung von Bereichen der Tasche verschlossen werden. Die Sensoranordnung 22a ist somit nicht fest mit der Busleitung 24a verbunden, sondern liegt lose auf der Busleitung 24a auf. Die Tasche 38 hält jedoch die Sensoranordnung 22a relativ zur Busleitung 24a in Position, sodass eine zuverlässige elektrische Kontaktierung der Busleitung 24a durch die Sensoranordnung 22a gewährleistet ist. Alternativ oder zusätzlich zu der Tasche 38 kann die Sensoranordnung 22a durch ein Band (nicht gezeigt) oder dergleichen an der Busleitung 24a gehalten werden. Durch den Verzicht auf eine feste elektrische Verbindung zwischen der Sensoranordnung 22a und der Busleitung 24a wird die Bekleidung 10 stark modular ausgebildet. Weitere Sensoranordnungen (nicht gezeigt) können ergänzt und/oder die bestehende Sensoranordnung 22a ausgetauscht werden.

**Fig. 4** zeigt eine vergrößerte Ansicht des Querschnitts der Bekleidung 10 entlang der Linie IV-IV in Fig. 3. Aus Fig. 4 ist ersichtlich, dass die Sensoranordnung 22a im Zwischenraum der Tasche 38 eingeklemmt ist. Die Sensoranordnung 22a weist dabei mehrere Kontaktflächen **42a, 42b, 42c, 42d** auf. Die Kontaktflächen 42a-42d dienen jeweils der elektrischen Verbindung mit elektrischen Leitungen **32a, 32b, 34a, 34b.**

Die elektrische Leitung 32a dient zur Bereitstellung einer Versorgungsspannung. Die elektrische Leitung 32b ist auf Masse geschaltet. Die Datenkommunikation mit der Sensoranordnung 22a erfolgt über die elektrischen Leitungen 34a, 34b. Die Datenkommunikation über die elektrischen Leitungen 34a, 34b erfolgt differentiell, d.h. gegenpolig. Hierdurch wird eine gegenüber externen Einflüssen resistente Datenübertragung gewährleistet.

Auf dem Trägerstreifen 26a kann zumindest ein redundantes Paar der elektrischen Leitungen 32a, 32b zur Spannungsversorgung angeordnet sein. Alternativ oder zusätzlich dazu kann auf dem Trägerstreifen 26a zumindest ein redundantes Paar der elektrischen Leitungen 34a, 34b zur Datenkommunikation angeordnet sein.

Die elektrischen Leitungen 32a, 32b, 34a und/oder 34b kann/können in Form einer elastischen Leitung/von elastischen Leitungen ausgebildet sein. Die elektrischen Leitungen 32a, 32b, 34a und/oder 34b können gewoben, gestrickt, gewirkt, gehäkelt und/oder genäht ausgebildet sein. Die elektrischen Leitungen 32a, 32b, 34a und/oder 34b kann können mit dem Trägerstreifen 26a verwoben, verstrickt, verwirkt, verhäkelt und/oder vernäht sein.

Zumindest eine der elektrischen Leitungen 32a, 32b, 34a, 34b kann eine ebene Kontaktfläche (nicht gezeigt) zur Kontaktierung der Sensoranordnung 22a aufweisen. Im Gegensatz zur schematischen Darstellung in Fig. 4 erfolgt die Kontaktierung der Sensoranordnung 22a bevorzugt in den Ecken einer Seitenfläche der Sensoranordnung 22a.

Die Sensoranordnung 22a weist zumindest einen Sensor auf. Im vorliegenden Fall weist die Sensoranordnung 22a einen Beschleunigungssensor **44a,** einen Drehratensensor **44b** und einen Magnetfeldsensor **44c** auf. Hierdurch wird eine sehr genaue Lageerfassung für eine virtuelle Körperdarstellung ermöglicht. Alternativ oder zusätzlich dazu kann die Sensoranordnung 22a einen Temperatursensor, einen Pulssensor und/oder einen Feuchtigkeitssensor aufweisen (alle nicht gezeigt).

**Fig. 5** zeigt eine Draufsicht auf einen Benutzer **46.** Der Benutzer 46 kann eine zuvor beschriebene Bekleidung 10 tragen. Alternativ oder zusätzlich dazu trägt der Benutzer 46 eine VR-Brille **48.** Die Auswerteeinheit 36 (siehe Fig. 2) kann in Form der VR-Brille 48 ausgebildet sein.

Vor dem Benutzer 46 befindet sich ein Gegenstand **50,** hier in Form eines Tisches. Auf dem Gegenstand 50 kann zumindest eine Nahfeldkommunikationseinheit, hier mehrere Nahfeldkommunikationseinheiten **52a, 52b, 52c** angeordnet, insbesondere aufgeklebt, oder ausgebildet sein. In der VR-Brille 48, beziehungsweise der zur VR-Brille 48 gehörenden Datenverarbeitungsvorrichtung (nicht gezeigt), können Informationen zu zumindest einer Nahfeldkommunikationseinheit 52a, 52b, 52c hinterlegt sein, die beim Annähern der VR-Brille 48 an die Nahfeldkommunikationseinheit 52a, 52b, 52c abgerufen werden. Die Informationen können über die VR-Brille 48 wiedergegeben werden. Hierdurch können dem Benutzer 46 Informationen zu dem Gegenstand 50 vermittelt werden, beispielsweise Informationen über die Kontur des Gegenstands 50. Weiterhin können dem Benutzer 46 Informationen vermittelt werden, die über den realen Gegenstand 50 hinausgehen.

Unter Vornahme einer Zusammenschau aller Figuren der Zeichnung betrifft die Erfindung zusammenfassend eine Bekleidung 10 in Form eines Multisensordresses mit mehreren Sensoranordnungen 22a-d, insbesondere zum Erfassen von Körperhaltungen und/oder Bewegungen eines Benutzers 46 der Bekleidung 10. Jede Sensoranordnung 22a-d weist mindestens einen Sensor 44a-c, insbesondere in Form einer inertial movement unit, auf. Die Sensoranordnungen 22a-d sind, insbesondere zueinander parallel geschaltet, über zumindest eine Busleitung 24a-d mit einer Datensammeleinheit 28 verbunden. Die Busleitung/en 24a-d kann/können jeweils einen Trägerstreifen 26a aus Stoff aufweisen. Die elektrische Verbindung der Sensoranordnungen 22a-d mit der jeweiligen Busleitung 24a-d erfolgt bevorzugt durch rein mechanisches Anpressen der Sensoranordnungen 22a-d auf die Busleitung 24a-d. Hierzu wird vorzugsweise ein Band und/oder eine Tasche 38 eingesetzt. Die Datensammeleinheit 28 weist bevorzugt ein Speichermittel und/oder ein Übertragungsmittel für die von den Sensoranordnungen 22a-d gemessenen Daten, aber keine Auswerteeinheit für diese Daten auf.

## Patentansprüche

1. Bekleidung (10) zur Datenerfassung, wobei die Bekleidung (10) folgendes aufweist:
a. Einen Grundkörper (20) aus einem flexiblen Material zum Bekleiden eines Benutzers (46) der Bekleidung (10);
b. mehrere Sensoranordnungen (22a-d) mit jeweils zumindest einem Sensor (44a-c) zur Erfassung von Zuständen des Benutzers (46);
c. eine Datensammeleinheit (28), die mit zumindest einigen Sensoranordnungen (22a-d) verbunden ist, um die von den Sensoranordnungen (22a-d) erfassten Daten zu sammeln;
d. wobeidie Bekleidung (10) eine Busleitung (24a-d) aufweist, auf der zumindest einige der Sensoranordnungen (22a-d) angeordnet sind, wobei die Busleitung (24a-d) mit der Datensammeleinheit (28) elektrisch verbunden ist, um von den Sensoranordnungen (22a-d) erfasste Daten an die Datensammeleinheit (28) zu übermitteln;
und
e. die Busleitung (24a-d) einen flexiblen Trägerstreifen (26a) mit mehreren flexiblen elektrischen Leitungen (32, 32a, 32b, 34, 34a, 34b) zur elektrischen Verbindung zumindest einiger Sensoranordnungen (22a-d) mit der Datensammeleinheit (28) aufweist
**dadurch gekennzeichnet, dass**
f. die Bekleidung eine Virtual Reality Brille (48) aufweist, die eine Datenverarbeitungsvorrichtung umfasst, die ausgebildet ist, Daten der Datensammeleinheit (28) zu empfangen und zu verarbeiten, wobei zumindest einige Sensoranordnungen (22a-22d) reversibel lösbar mit der Busleitung (24a-d) verbunden sind und auf der Busleitung (24a-d) lose aufliegen, wobei die elektrische Anbindung der Sensoranordnungen (22a-d) ausschließlich durch mechanisches Anpressen der Sensoranordnungen (22a-d) auf die Busleitung (24a-d) erfolgt, wobei die Sensoranordnungen (22a-d) in einer flexiblen Tasche (38) und/oder unter einem flexiblen Band der Bekleidung (10) aufgenommen sind, wobei die Tasche und/oder das Band dazu ausgebildet ist, den Anpressdruck zu erzeugen.

2. Bekleidung nach Anspruch 1, bei der die Busleitung (24a-d) in Form einer parallelen Busleitung (24a-d) ausgebildet ist, sodass zumindest einige Sensoranordnungen (22a-d) unabhängig voneinander elektrisch mit der Datensammeleinheit (28) verbunden sind.

3. Bekleidung nach Anspruch 1 oder 2, bei der der Trägerstreifen (26a) auf den Grundkörper (20) genäht ist.

4. Bekleidung nach einem der vorhergehenden Ansprüche, bei der der Grundkörper (20) und/oder der Trägerstreifen (26a) aus Stoff ausgebildet ist/sind.

5. Bekleidung nach einem der vorhergehenden Ansprüche, bei der die Datensammeleinheit (28) reversibel lösbar mit der Busleitung (24a-d) verbunden ist.

6. Bekleidung nach Anspruch 5, bei der die elektrische Anbindung der Datensammeleinheit (28) ausschließlich durch mechanisches Anpressen der Datensammeleinheit (28) auf die Busleitung (24a-d) erfolgt.

7. Bekleidung nach einem der vorhergehenden Ansprüche, bei der die Datensammeleinheit (28) in einer flexiblen Tasche (38) und/oder unter einem flexiblen Band der Bekleidung (10) aufgenommen ist.

8. Bekleidung nach einem der vorhergehenden Ansprüche, bei der die Bekleidung (10) mehrere Busleitungen (24a-d) aufweist, die in zumindest einem Knotenpunkt der Bekleidung (10) zusammengeführt sind.

9. Bekleidung nach einem der vorhergehenden Ansprüche, bei der die Bekleidung (10) einen Nahfeldkommunikationssender und einen Nahfeldkommunikationsempfänger aufweist, um zumindest eine Nahfeldkommunikationseinheit (52a-c) in der Umgebung der Bekleidung (10) zu detektieren.

10. Bekleidung nach Anspruch 9, **dadurch gekennzeichnet, dass** der Nahfeldkommunikationsempfänger einen Teil der Datenverarbeitungsvorrichtung der Virtual Reality Brille (48) darstellt.

11. Verwendung einer Bekleidung (10) nach einem der vorhergehenden Ansprüche, bei der die Bekleidung (10) zur Programmierung des Bewegungsablaufs eines Roboters eingesetzt wird.

## Claims

1. Garment (10) for data detection, wherein the garment (10) has:
a. a main body (20) made of a flexible material for clothing a user (46) of the garment (10);
b. several sensor arrangements (22a-d), each with at least one sensor (44a-c) for detecting conditions of the user (46);
c. a data collection unit (28) connected to at least some sensor arrangements (22a-d) for collecting the data detected by the sensor arrangements (22a-d);
d. wherein the garment (10) has a bus line (24a-d) on which at least some of the sensor arrangements (22a-d) are arranged, wherein the bus line (24a-d) is electrically connected to the data collection unit (28) in order to transmit data detected by the sensor arrangements (22a-d) to the data collection unit (28);
and
e. the bus line (24a-d) has a flexible carrier strip (26a) with several flexible electrical lines (32, 32a, 32b, 34, 34a, 34b) for electrically connecting at least some sensor arrangements (22a-d) to the data collection unit (28),
**characterized in that**
f. the garment has a virtual reality headset (48) which comprises a data processing device which is designed to receive and process data from the data collection unit (28), wherein at least some sensor arrangements (22a-22d) are reversibly detachably connected to the bus line (24a-d) and rest loosely on the bus line (24a-d), wherein the electrical connection of the sensor arrangements (22a-d) takes place exclusively by mechanically pressing the sensor arrangements (22a-d) onto the bus line (24a-d), wherein the sensor arrangements (22a-d) are accommodated in a flexible pocket (38) and/or under a flexible band of the garment (10), wherein the pocket and/or the band is designed to generate the contact pressure.

2. Garment according to claim 1, wherein the bus line (24a-d) is designed in the form of a parallel bus line (24a-d) so that at least some sensor arrangements (22a-d) are electrically connected to the data collection unit (28) independently of one another.

3. Garment according to claim 1 or 2, wherein the carrier strip (26a) is sewn onto the main body (20).

4. Garment according to any one of the preceding claims, wherein the main body (20) and/or the carrier strip (26a) is/are made of fabric.

5. Garment according to any one of the preceding claims, wherein the data collection unit (28) is reversibly detachably connected to the bus line (24a-d).

6. Garment according to claim 5, wherein the electrical connection of the data collection unit (28) takes place exclusively by mechanically pressing the data collection unit (28) onto the bus line (24a-d).

7. Garment according to any one of the preceding claims, wherein the data collection unit (28) is accommodated in a flexible pocket (38) and/or under a flexible band of the garment (10).

8. Garment according to any one of the preceding claims, wherein the garment (10) has several bus lines (24a-d) which are brought together in at least one node of the garment (10).

9. Garment according to any one of the preceding claims, wherein the garment (10) has a near-field communications transmitter and a near-field communications receiver in order to detect at least one near-field communications unit (52a-c) in the vicinity of the clothing (10).

10. Garment according to claim 9, **characterized in that** the near-field communications receiver is part of the data processing device of the virtual reality headset (48).

11. Use of a garment (10) according to any one of the preceding claims, wherein the article of clothing (10) is used for programming the movement sequence of a robot.

## Revendications

1. Vêtement (10) dévolu à la saisie de données, lequel vêtement (10) comprend les éléments suivants :
a. un corps de base (20) en un matériau flexible, conçu pour habiller un utilisateur (46) dudit vêtement (10) ;
b. plusieurs ensembles de détection (22a-d), chacun étant respectivement équipé d'au moins un capteur (44a-c) en vue de saisir des états de l'utilisateur (46) ;
c. une unité (28) collectrice de données, connectée à au moins quelques ensembles de détection (22a-d) en vue de collecter les données saisies par lesdits ensembles de détection (22a-d) ;
d. ledit vêtement (10) étant muni d'une ligne de bus (24a-d) sur laquelle sont disposés au moins quelques-uns des ensembles de détection (22a-d), ladite ligne de bus (24a-d) étant raccordée électriquement à l'unité (28) collectrice de données de manière à transmettre, à ladite unité (28) collectrice de données, des données saisies par lesdits ensembles de détection (22a-d) ;
et
e. ladite ligne de bus (24a-d) étant dotée d'un ruban flexible de support (26a) comptant plusieurs lignes électriques flexibles (32, 32a, 32b, 34, 34a, 34b), en vue du raccordement électrique d'au moins quelques ensembles de détection (22a-d) à l'unité (28) collectrice de données,
**caractérisé par le fait que**
f. ledit vêtement est pourvu de lunettes (48) de réalité virtuelle, incluant un dispositif de traitement de données affecté à la réception et au traitement de données de l'unité (28) collectrice de données, sachant qu'au moins quelques ensembles de détection (22a-22d) sont connectés à la ligne de bus (24a-d) de façon réversible et libérable, et reposent avec jeu sur ladite ligne de bus (24ad), le raccordement électrique des ensembles de détection (22a-d) étant effectué, de manière exclusive, en pressant mécaniquement lesdits ensembles de détection (22a-d) sur ladite ligne de bus (24a-d), lesquels ensembles de détection (22a-d) sont intégrés dans une poche souple (38) et/ou au-dessous d'un ruban flexible du vêtement (10), ladite poche et/ou ledit ruban étant conçu(e) pour engendrer la pression de contact.

2. Vêtement selon la revendication 1, dans lequel la ligne de bus (24a-d) est réalisée sous la forme d'une ligne de bus (24a-d) parallèle, de telle sorte qu'au moins quelques ensembles de détection (22a-d) soient raccordés électriquement à l'unité (28) collectrice de données indépendamment les uns des autres.

3. Vêtement selon la revendication 1 ou 2, dans lequel le ruban de support (26a) est cousu sur le corps de base (20).

4. Vêtement selon l'une des revendications précédentes, dans lequel le corps de base (20) et/ou le ruban de support (26a) consiste(nt) en du tissu.

5. Vêtement selon l'une des revendications précédentes, dans lequel l'unité (28) collectrice de données est connectée à la ligne de bus (24a-d) de façon réversible et libérable.

6. Vêtement selon la revendication 5, dans lequel le raccordement électrique de l'unité (28) collectrice de données est effectué, de manière exclusive, en pressant mécaniquement ladite unité (28) collectrice de données sur la ligne de bus (24a-d).

7. Vêtement selon l'une des revendications précédentes, dans lequel l'unité (28) collectrice de données est intégrée dans une poche souple (38) et/ou au-dessous d'un ruban flexible dudit vêtement (10).

8. Vêtement selon l'une des revendications précédentes, ledit vêtement (10) comprenant plusieurs lignes de bus (24a-d) rassemblées en au moins un point nodal dudit vêtement (10).

9. Vêtement selon l'une des revendications précédentes, ledit vêtement (10) comprenant un émetteur de communication en champ proche et un récepteur de communication en champ proche, afin de détecter au moins une unité (52a-c) de communication en champ proche dans l'environnement dudit vêtement (10).

10. Vêtement selon la revendication 9, **caractérisé par le fait que** le récepteur de communication en champ proche représente une partie du dispositif de traitement de données des lunettes (48) de réalité virtuelle.

11. Utilisation d'un vêtement (10) conforme à l'une des revendications précédentes, ledit vêtement (10) étant employé pour programmer la séquence de mouvements d'un robot.
